# EUROPEAN PATENT APPLICATION

(11) **EP 4 042 932 A1**
(43) Date of publication of application: **17.08.2022**
(21) Application number: 20873455.8
(22) Date of filing: 09.10.2020
(51) Int. Cl.: A61B 5/00, A61B 5/16

(54) **ELECTROMAGNETIC BIG DATA REMOTE SENSING OF PHYSIOLOGICAL DYNAMICS**

(30) Priority: 09.10.2019 CN 201910953194
(71) Applicant: Liu, Fenghua, Guiyang, Guizhou 550025 (CN)
(72) Inventor: Liu, Fenghua, Guiyang, Guizhou 550025 (CN)
(74) Representative: Ipey
(86) International application number: PCT/CN2020/000236
(87) International publication number: WO 2021/068430

(57) **Abstract**

Electromagnetic big data remote sensing of the physiological dynamics. The related technology relates to the field of physiological or psychological remote sensing. The technical problem to be solved is performing remote sensing on the physiological or psychological dynamics related to micro and macro electromagnetic media such as nucleons, electrons, atoms, molecules, cells, tissues, organs, and bones of humans, and individual animals and plants at the approximate speed of light regardless of day and night, rain, snow, and fog. Reflected waves and scattered waves generated by applying electromagnetic waves in the frequency band range of 0.3 Hz to 10 THz to irradiate biological individuals can represent the electromagnetic dynamics thereof. An electromagnetic dynamics database of organisms can be built by performing analog-to-digital conversion on information of the reflected waves and the scattered waves. The reflected waves and the scattered waves extracted after irradiating the organisms with the land, sea, and air emission sources can be compared and filtered by the database to realize the qualitative, semi-quantitative, and quantitative 24-hour all-weather physiological or psychological dynamics of the biological individuals on a local or global scale. The main use relates to driving under the influence, fatigue driving, self-driving, big data, large ecology, artificial intelligence, etc.

## Description

(I) TECHNICAL FIELD: The technical scheme claimed for protection belongs to the field of remote sensing of physiology or psychology.

(II) BACKGROUND: When humans, animals and plants are irradiated with visible light, which is an electromagnetic wave, physical phenomena occur such as reflection, scattering, refraction, absorption, transmission and diffraction. Reflected waves and scattered waves of visible light are generally used to extract characteristic information of colors of biological surficial electromagnetics. Data clusters generated by the analog-to-digital conversion of information of reflected waves and scattered waves can be used to achieve qualification, semi-quantification and quantification of colors of surficial electromagnetics of biological individuals. Therefore, technically speaking, big data remote sensing of colors of biological surficial electromagnetics can be achieved by the technology of reflected waves and scattered waves of visible light.

(III) SUMMARY: The technical problem to be solved by the present invention is remote sensing of the physiological or psychological dynamics of an individual of humans, animals and plants at nearly the speed of light for 24 hours a day, day and night and regardless of rain, snow and fog. The invention can be used in various aspects including driving under the influence (DUI), fatigue driving, autonomous driving, big data, big ecology, artificial intelligence, etc. Physiological media such as nucleons, electrons, atoms, molecules, ions, water, body fluids, blood, DNA, RNA, amino acids, sugars, fats, proteins, cells, tissues, organs, and bones construct biological individuals on a microscopic to macroscopic level, while essentially demonstrating the microscopic and macroscopic electromagnetic nature of the biological individuals comprising nucleons and electrons of an astronomical order of magnitude. All physiological dynamics related to biochemistry and biophysics are essentially electromagnetic fluctuations. Sensory stimuli to eyes, ears, nose, tongue, body and mind triggered by internal psychological factors such as joy, anger, melancholy, anxiety, sorrow, fear and fright, or external environmental factors will all induce electromagnetic fluctuations of biological individuals accordingly. Terahertz waves, millimeter waves, centimeter waves, decimeter waves, meter waves, radio frequency waves and low-frequency waves, with wavelengths ranging from a level of sub-millimeter to a level of 100 million meters, can all interact with biological electromagnetics. The penetration depth, action medium and nature of action of the electromagnetic waves of different wavelengths at the biological interface change accordingly with the wavelength. The reflected wave, scattered wave, refracted wave, absorbed wave, transmitted wave or diffracted wave obtained by irradiating a biological individual with single-frequency, narrow-band or wide-band electromagnetic waves in a frequency range of 0.3 Hz - 10 THz can characterize electromagnetic nature and strength relevant to the physiological or psychological media inside the biological individual such as nucleons, electrons, atoms, molecules, ions, water, body fluids, blood, DNA, RNA, amino acids, sugars, fats, proteins, cells, tissues, organs and bones. Generally, data clusters generated by analog-to-digital conversion of reflected waves and scattered waves are used to characterize the biological electromagnetics of an individual of humans, animals and plants, and comparison of the data clusters can be used to realize remote-sensing detection of the physiological or psychological dynamics of a biological individual. The data of electromagnetic reflected waves and scattered waves of living organisms is a three-dimensional massive dynamic data cluster, wherein some of the data are relatively stable, demonstrating conservatism, while some other data show greater variability. For high-concern targets such as drivers, the data can be updated every short period of time for remote sensing of alcohol content or fatigue of the driver such as to alert and ensure life and property safety. On the contrary, a longer period for data update can be set as required for general-concern targets. The effects of irradiation and sampling with limited power density and very short action time on humans, animals and plants are ignorable, and hence the present invention is technically safe. The penetration depth of visible light with a wavelength of about 0.35 µm to 0.75 µm is smaller than 0.75 µm at the biological interface. Limited by the nature of the wavelength of visible light, the physiological information of generally opaque organisms extracted by visible light is surficial data thereof. Meanwhile, the qualitative capability of visible light is mainly manifested in color identification, while the application value of color data of biological surficial electromagnetics is very limited in physiology. Additionally, weather like rain, snow and fog has a substantial interference with the visible light technology because of the wavelength of visible light. Further, the plane angle and elevation angle of incidence of visible light are restricted for extraction of characteristic facial information relevant to psychological dynamics, and characteristic information related to psychology cannot be collected beyond a certain angle. In contrast, the electromagnetic band with wavelengths ranging from a level of sub-millimeter to a level of 100 million meters can extract three-dimensional electromagnetic information from the surface to the inside of a biological individual. While visible light can only extract data of color characterized by seven colors, i.e., red, orange, yellow, green, blue, indigo and violet, of a sub-micrometer depth at the biological interface, the electromagnetic band with wavelengths ranging from a level of sub-millimeter to a level of 100 million meters can extract both surficial and internal electromagnetic data of non-visible light nature at varied depths ranging from a level of sub-millimeters to a level of meters. Therefore, volume of data collected by electromagnetic waves in a frequency range of 0.3 Hz - 10 THz can theoretically exceed that by visible light by 1 million times. Meanwhile, different wavelengths lead to difference in properties of the electromagnetic data thereof. Electromagnetic waves with longer wavelengths than visible light and stronger capability against interference by rain, snow and fog can extract electromagnetic information of humans, animals and plants at varied observation angles, day and night, and regardless of rain, snow and fog for remote sensing of physiological or psychological dynamics. Therefore, the present invention is significantly advanced compared to the most comparable prior art of visible light photography.

(IV) DETAILED DESCRIPTION: Although individuals of humans, animals and plants are diverse in their outlooks, the essence thereof is electromagnetic structures comprising nucleons and electrons of an astronomical order of magnitude and of different properties, quantities and distributions. All of the biological physiology, psychology and external environmental influences are essentially manifested as electromagnetic fluctuations, and the relevant electromagnetic information in the frequency range of 0.3 Hz - 10 THz can be extracted from the reflected waves and scattered waves obtained by irradiating a biological individual with electromagnetic waves with wavelengths ranging from a level of sub-millimeters to a level of 100 million meters. Application of UV, X-ray and gamma rays at higher frequencies is restricted due to the high energy thereof which may damage organisms. The biological electromagnetic dynamics corresponding to physiological, psychological and external environment influences can be divided into continuous fluctuations and significant switch-type changes. The physiological electromagnetic dynamics corresponding to wakefulness, sleep and exercise must be significant switch-type changes. The psychological electromagnetic dynamics induced by the seven emotions, i.e., joy, anger, melancholy, anxiety, sorrow, fear and fright, are also significantly different compared with that induced by calmness. Environmental factors such as day, night, rain, drought, temperature rise and snow may induce significant changes in the physiological electromagnetic dynamics of a plant. The harm caused by driving under the influence (DUI) is widely known. The alcohol content is usually determined by a method of breath alcohol test or blood test, the principle of which is to detect the gaseous or liquid ethanol molecule CH₃CH₂OH in a gas or liquid mixture. It is not only slow with this method, but inspection volume thereby is insignificant compared with the traffic flow, and it interferes with the normal traffic flow of non-DUIs. The physiological structure of a human body is essentially a mixed electromagnetic structure of gas, liquid and solid. Once ethanol molecules enter a human body, a portion of the molecules spread and circulate in the body in a free state, while a second portion of molecules react with corresponding components to produce a series of biochemical products. Meanwhile, ethanol molecules and the series of biochemical products are metabolized by relevant functions and gradually discharged from the body. There is no doubt that the physiological electromagnetic changes induced by ethanol molecules can be characterized by electromagnetic waves in a relevant frequency range of 0.3 Hz - 10 THz, and hence it is theoretically feasible to develop a remote sensing that specifically detects DUI against the electromagnetic commonality of ethanol molecules and the series of biochemical products thereof based on the sensitivity and selectivity of an electromagnetic wave probe to lower the cost and facilitate the application, instead of solely relying on comparisons of electromagnetic information of individual driver's drinking state and non-drinking state. Fatigue driving has raised widespread attention worldwide, and although one knows exactly whether he/she is fatigued, the scientific and technological community is not convinced yet on how to qualify and quantify fatigue. However, fatigue can be quantified and qualified by characterizing the dynamic changes in the electromagnetic structure of a human body with electromagnetic scattered waves and reflected waves in the relevant frequency range of 0.3 Hz - 10 THz. A driver's mental state is constantly changing during driving, from a state of freshness, to being alright, to being tired and to drowsiness. The remote sensing equipment arranged at each intersection can extract the reflected waves and scattered waves of electromagnetics of a driver in the relevant frequency range of 0.3 Hz - 10 THz. The frequency spectrum in a relevant sensitive frequency range can show the continuous fluctuations and significant switch-type changes thereof. If the data indicates that the driver is in a state of tiredness or drowsiness, the loudspeaker preset in the driver's cab will be turned on for emergency sensory stimulations, and moreover, the driver is requested to pull over to a safe side of road or drive to a service area to rest so as to avoid any loss of life and property. Autonomous driving is a technology and business hot spot worldwide. By monitoring relevant dangerous physiological and psychological states such as DUI, fatigue driving and road rage driving at the speed of light for early intervention of warning and avoidance, relevant remote sensing technology can provide reliable information assurance conducted at nearly the speed of light for the development of autonomous driving. The azimuth and elevation angles of an emission light source and a sensor of the reflected waves and scattered waves relative to the position of a biological individual in a rectangular coordinate system or a spherical coordinate system are very important for the comparability of the data. The data extracted at different azimuth and elevation angles vary without comparability. The azimuth and elevation angles can be determined according to existing remote sensing sites which are indoor, at intersections, at a height, at sea, underwater, on the seabed, in the sky or on a satellite, or by planning a new remote sensing site. Once the azimuth and elevation angles are determined, the data of reflected waves and scattered waves for different physiological and psychological states of a biological individual extracted in a laboratory, on a ground base, in a sky base or in a space base are comparable, such that a real-time monitoring of the dynamic changes of the biological individual can be achieved. Individuals of humans, animals and plants are electromagnetic structures comprising an astronomical order of magnitude of nucleons and electrons of different properties, quantities and distributions based on dozens of elements. Each organism has a different electromagnetic structure, and hence the electromagnetic reflected waves and scattered waves of each organism also vary. Therefore, the electromagnetic reflected waves and scattered waves extracted by the technology of the present invention can be used for real-time remote sensing and monitoring of the physiological or psychological dynamics of an individual of humans, animals and plants. Relevant electromagnetic big data can be applied through artificial intelligence in aspects like DUIs, fatigue driving, autonomous driving and big ecology of animals and plants in not only China, but countries along the "Belt and Road" and a community of common destiny.

## Claims

1. Preamble: a subject title of the claimed invention is "REMOTE SENSING OF PHYSIOLOGICAL DYNAMIC ELECTROMAGNETIC BIG DATA"; the closest current technology is the visible light photography technology; the reflection-scattering wave obtained by irradiating human, animals and plants with visible light can represent the electromagnetic color features of biological surface; and the common feature of the present invention and the visible light photography technology is using electromagnetic waves as detection tools;

2. features: the visible light photography technology in the 0.35µm-0.75µm wavelength range is seriously interfered by rain, snow and fog due to the wavelength; and the incident angle and elevation angle of visible light for extracting the psychological information related to the emotions of joy, anger, worries, anxiousness, sadness, fear, and scare are limited; it is impossible to collect enough psychological information when the incident angle and elevation angle are beyond certain values; because of the wavelength limitation of the visible light, the penetration depth of the visible light at the biological interface is less than 0.75µm; so the physiological information extracted by visible light is the surface data of the usually opaque organisms, and the qualitative ability of visible light is mainly manifested in color discrimination; while the superficial color data have very limited physiological applications; the technical feature of the present invention is that the electromagnetic waves in the wavelength range of 30 µm-100 million meters including terahertz wave, millimeter wave, centimeter wave, decimeter wave, meter wave, radio frequency wave and low frequency wave are used to irradiate human, animal and plant individuals, then the reflection-scattering wave information is extracted to represent the electromagnetic dynamic information of physiological or psychological media of astronomical nucleon-electron assemblies constructed from micro to macro, such as the nucleons, electrons, atoms, molecules, ions, water, body fluid, blood, DNA, RNA, amino acids, sugars, fats, proteins, cells, tissues, organs, bones, fruits, flowers, leaves, stems, bark, stems and roots; nucleons, electrons, atoms and molecules are all microscopic electromagnetic assemblies of a physiological medium; three-dimensional assembly of the massive electromagnetic assemblies with different properties, quantities and distributions can determine the different specificity of the electromagnetic structure of any human, animals and plants; the electromagnetic coefficients such as electrical conductivity σ, relative permittivity εᵣ and magnetic susceptibility χm related to the nucleon-electron and atom-molecule clusters of various psychological media such as skin, muscle, fat, body fluids, blood, blood vessels, organs, bones, fruits, flowers, leaves, stems, bark, stems and roots are different from each other; the linear equations such as *J_{c}*=(*σE, D*=*εᵣ-ε₀E* and *M*=*χₘH* show that different electromagnetic coefficients lead to different electromagnetic energy conversion efficiencies; the non-visible electromagnetic waves in the wavelength range of 30 µm-100 million meters can irradiate nucleon-electron and atom-molecule clusters of human, animal and plant and cause various strong-weak electromagnetic interactions with nucleon-electron and atom-molecule clusters such as vibration, rotation, polarization, magnetization or conduction at different frequencies; in the meantime, the change of electromagnetic energy is related to the electromagnetic coefficients such as electrical conductivity σ, relative dielectric constant εᵣ and magnetic susceptibility χₘ of psychological media; therefore, the spectrum features revealed by the interaction between different atom-molecular electromagnetic microunit clusters of any human, animals and plants and electromagnetic waves in the wavelength range of 30 µ m-100 million meters are different from each other; the human and living animals and plants built by the astronomical nucleon-electron and atom-molecule electromagnetic assembly have not only small electromagnetic fluctuations but also significant electromagnetic fluctuations affected by factors such as sleep, wakefulness, exercise, drinking, fatigue, anger, drought, flood, cold, heat, or day and night; the physiological or psychological state of human, animals and plants can be qualitatively and quantitatively determined by measuring the small and significant electromagnetic fluctuations of the nucleon-electron and atom-molecule clusters; the electromagnetic waveband in the wavelength range of 30 µm-100 million meters whose wavelengths expand from micrometer to one hundred million meters can extract the exterior-interior electromagnetic data of non-visible light properties covering partial or whole electromagnetic media of humans, animals and plants at various depths from surface to the depth of less than one hundred megameter through reflection waves, scattering waves, refraction waves, absorption waves, transmission waves or diffraction waves; reflection, scattering, refraction, absorption, transmission or diffraction can occur back and forth for a plurality of times on multiple layers of skins, muscles, fats, body fluids, blood, blood vessels, organs, bones, fruit, flowers, leaves, stems, bark, trunks and roots. Considering different thickness of the various physiological layers on a level of millimeter, centimeter, decimeter, meter or even larger, and the ultra-fast propagation speed of electromagnetic waves, the electromagnetic information extracted by multiple back-and-forth reflection, scattering, refraction, absorption, transmission or diffraction at various physiological layers after irradiating a living body of humans, animals and plants with femtosecond pulsed electromagnetic waves can be **characterized by** the time domain spectrum of electromagnetic intensity on a time scale of picosecond or nanosecond. The time domain signal is converted by the Fourier transform into a sine wave, and then parameters like frequency, amplitude and phase are used to characterize the electromagnetic information of each of the three-dimensional surficial and internal layers to achieve quantification and qualification of time domain data of physiological or psychological dynamics; under the standard working parameters determined by experiments, the electromagnetic wave scanning in the waveband of 30µm-1000 million meters is used to extract the reflection-scattering spectrum or absorption spectrum of human body and various animals and plants; reflection-scattering spectrum troughs or absorption spectrum crests, which can show sensitive wavelengths or wavebands where non-visible light electromagnetic wavebands interact strongly with nucleon-electron and atom-molecule clusters of human, specific animals and plants; the single wavelength or waveband with the strongest electromagnetic interaction is chosen as the sensitive detection wave; the detection waves with sensitive wavelength or waveband are used to irradiate the human body and specific species of animals and plants; then, light intensity distribution of the reflection-scattering waveat the specific interface depth is obtained the distribution of luminous intensity of reflected waves and scattered waves can be transformed into one, two or more individual-specific tens of millions of two-dimensional pixel matrix can be obtained according to the light intensity distribution obtained by analog-to-digital conversion; the physiological or psychological dynamic changes such as sleep, wakefulness, exercise, drinking, fatigue, anger, drought and flood, cold and heat, day and night can be quantified and qualified by comparing the changes in digitals in the digital matrices or digital matrix blocks of the individual of humans, animals or plants of different physiological or psychological states. The reflected and scattered luminous intensity extracted by irradiating humans and various animals and plants or parts thereof with femtosecond pulsed electromagnetic waves of each wavelength or a band of between 30 µm and 1000 million meters is rapidly attenuated in a time scale of picosecond or nanosecond. The time domain luminous intensity integration is mapped against the wavelength range of 30 µm - 1000 million meters. The wavelength or band corresponding to one, two or more troughs is the characteristic wavelength or band where the electromagnetic waves of the band between 30 µm and 1000 million meters interacts strongly with humans and various animals and plants. Femtosecond pulsed electromagnetic waves of characteristic wavelength or band are used to irradiate individuals of humans and various animals and plants or parts thereof to extract the reflected and scattered time domain spectrum, which undergoes Fourier transform such that the electromagnetic fluctuations of various sizes of the clusters of the nucleons, electrons, atoms and molecules can be **characterized by** parameters like frequency, amplitude and phase of sine waves. The physiological and psychological dynamic changes of individuals of humans, animals and plants can be quantified and qualified by comparing the frequency, amplitude and phase of sine waves under different physiological, psychological or environmental states such as sleep, wakefulness, exercise, drinking, fatigue, anger, drought, flood, cold and heat, and day and night. Both frequency domain spectrum and time domain spectrum measure electromagnetic fluctuations of various sizes of three-dimensional surficial and internal clusters of nucleon, electrons, atoms and molecules. The sensitivity of these two spectral analyses is different. The frequency domain and the time domain are different in nature with their own focus each, but they can be mutually corroborated; the electromagnetic band whose wavelengths expand from micrometer to one hundred million meters can extract the exterior-interior electromagnetic data of non-visible light properties covering partial or whole electromagnetic media of of humans, animals and plants at various depths from surface to the depth of hundreds of meters; therefore, the electromagnetic wave in the waveband of 30µm-1000 million meters differs from that by visible light in an exponential order of magnitude and has different data properties due to the different wavelengths; the electromagnetic waves with longer wavelengths and better resistance to rain, snow and fog than visible light can extract bio-electromagnetic information for remote sensing of physiological and psychological dynamics at different observation angles regardless of day and night and rain, snow and fog weather; different from the visible light photography technology, the advanced technical features of the present invention, such as time domain spectral Fourier transformed data three-dimensional exterior-interior electromagnetic information, non-visible light waveband physiological and psychological remote sensing, resistance to rain-snow-fog interference, and no observation angle limitation, are based on relevant electromagnetic scientific theories, so, the advantage of the present invention is remarkable compared with the closest existing visible light photography technology; the electromagnetic structures of the three-dimensional surficial and internal clusters of nucleons, electrons, atoms and molecules as physiological or psychological media of individuals of humans, animals and plants are different from each other. Meanwhile, induced by physiological or psychological changes of individuals of humans, animals and plants, the clusters of nucleons, electrons, atoms and molecules thereof all demonstrate electromagnetic fluctuations of various sizes every second, day and night; electromagnetic wave in the waveband of 30µm-1000 million meters is used to scan and extract the reflection-scattering spectrum of a specific animal kind in normal physiological state; the sensitive wavelength or waveband of the characteristic wave trough can irradiate the individual to represent the small and significant electromagnetic fluctuations of the three-dimensional exterior-interior nucleon-electron and atom-molecule clusters by the electromagnetic frequency domain and time domain data ; various physiological or psychological states of human such as sleep, wakefulness, exercise, drinking, fatigue, anger, drought, flood, cold and heat, day and night, etc. can be qualitatively, and quantitatively determined by comparing the fluctuations of the individual electromagnetic index; so the technical scope of the claimed invention is method, which first applies single wavelength, two or more wavelengths or wide waveband or narrow waveband electromagnetic waves with spectral features related to different physiological or psychological states of a specific kind of objects to irradiate human body to extract reflection waves, scattering waves, refraction waves, absorption waves, transmission waves or diffraction waves in different physiological or psychological statesthrough electromagnetic interaction to characterize electromagnetic information related to a biological medium like nucleons, electrons, atoms, molecules, ions, water, body fluids, blood, DNA, RNA, amino acids, sugars, fats, proteins, cells, tissues, organs, bones, fruit, flowers, leaves, stems, bark, trunks and roots in humans, animals and plants for qualitative and quantitative remote sensing of physiological or psychological dynamics.
